# EUROPÄISCHE PATENTSCHRIFT

(11) **EP 2 401 014 B1**
(45) Veröffentlichungstag und Bekanntmachung des Hinweises auf die Patenterteilung: **06.11.2013**
(21) Anmeldenummer: 10704574.2
(22) Anmeldetag: 19.02.2010
(51) Int. Cl.: A61M 11/00, A61M 15/00, B05B 17/06

(54) **INHALATIONSTHERAPIEVORRICHTUNG**
INHALATION THERAPY DEVICE
DISPOSITIF DE THÉRAPIE PAR INHALATION

(30) Priorität: 20.02.2009 DE 102009001037
(43) Veröffentlichungstag der Anmeldung: 04.01.2012
(73) Patentinhaber: PARI Pharma GmbH, 82319 Starnberg (DE)
(72) Erfinder: ACHTZEHNER, Wolfgang, Alling 82239 (DE)
(74) Vertreter: HOFFMANN EITLE
(86) Internationale Anmeldenummer: PCT/EP2010/052128
(87) Internationale Veröffentlichungsnummer: WO 2010/094767

(56) Entgegenhaltungen:
- EP-A1- 0 635 312
- WO-A1-2004/004813
- WO-A1-2004/039442
- WO-A1-2008/117264
- US-A1- 2003 196 660
- US-A1- 2009 241 948

## Beschreibung

Die Erfindung betrifft eine Inhalationstherapievorrichtung mit einem Membranaerosolgenerator für die Vernebelung einer therapeutisch wirksamen Flüssigkeit.

Für die Vernebelung von Flüssigkeiten zur Inhalationszwecken werden verschiedene Aerosolgeneratoren eingesetzt. Beispielsweise kommen Düsenvernebler oder Ultraschallvernebler zum Einsatz, die aber im Vergleich mit Membranaerosolerzeugern der hier in Rede stehenden Art eine lange Einschaltphase (> 1s) aufweisen, bevor ein Aerosol erzeugt und abgegeben wird.

WO 2004/004813 A beschreibt eine Inhalationstherapievorrichtung, bei der die Temperatur der zu vernebelnden Flüssigkeit erfasst wird und die Steuerung der Verneblung derart stattfindet, dass die Verneblung nur in bestimmten Zeitintervallen erfolgt, deren Länge und/oder Häufigkeit in Abhängigkeit von der erfassten Temperatur von der Steuereinrichtung festgelegt wird.

WO 2004/039442 A1 beschreibt eine Inhalationstherapievorrichtung, bei der mindestens zwei unterschiedliche Schwingungsfrequenzen (f1 und f2) für die Membrane erzeugt werden und der Membrane alternierend zugeführt werden, wobei es sich bei f1 um die Ansteuerfrequenz handelt, um die Membrane in Schwingungen zu versetzen, und bei f2 um eine Frequenz zur Bestimmung eines Betriebszustandes, die also lediglich Messzwecken dient.

US 2003/196660 A1 beschreibt eine Vorrichtung mit der ein Aerosol in einen Gasstrom abgegeben werden soll. In dieser Vorrichtung wird dabei in der Einatmungsphase die Spannung eines piezoelektrischen Elements derart erhöht, dass eine Verneblung stattfindet. In der Ausatmungsphase wird die Spannung des piezoelektrischen Elements dann verringert1 oder teilweise ganz ausgeschaltet, so dass keine Verneblung stattfindet.

EP 0 635 312 A1 beschreibt einen Ultraschallzerstäuber, bei dem ein Übergang in den automatischen, intermittierenden Modus dadurch erzielt wird, dass der Nutzer einen Bedienschalter dreimal für jeweils weniger als 4 Sekunden einschaltet.

WO 2008/117264 A1 und US 2009/0241948 beschreiben einen Befeuchter zur Befeuchtung der Luftröhre mit sterilem Wasser oder einer Kochsalzlösung.

Aus WO 93/10910 A ist ein Membranaerosolerzeuger bekannt, der eine Membran und eine Schwingungserzeugungseinrichtung umfasst. Bei der Schwingungserzeugungseinrichtung handelt es sich um einen Piezoschwinger, dem ein Ansteuersignal zugeführt wird, durch das der Piezoschwinger in Schwingungen versetzt wird. Da der Piezoschwinger mit der Membran verbunden ist, wird auch diese in Schwingungen versetzt, so dass eine auf der einen Seite der Membran anstehende Flüssigkeit durch die Öffnungen in der Membran hindurchgefördert und auf der anderen Seite der Membran in Form eines Aerosols abgegeben wird. Bei dem Ansteuersignal für den Piezoschwinger handelt es sich um eine Wechselspannung, die von einer Ansteuerschaltung an den Piezoschwinger des Aerosolgenerators angelegt wird, wenn ein Aerosol erzeugt werden soll.

Membranaerosolgeneratoren dieser Art werden vorteilhaft in Inhalationstherapieverneblern eingesetzt, da der Aerosolgenerator mit Hilfe der Ansteuerschaltung leicht ein- bzw. ausgeschaltet werden kann. In diesem Zusammenhang werden die Zeiträume als Einschaltphasen des Aerosolgenerators bezeichnet, in denen dem Aerosolgenerator des Inhalationstherapieverneblers ein Ansteuersignal zugeführt wird. Dabei werden herkömmlicherweise zwei Betriebsarten unterschieden.

Bei einem kontinuierlich arbeitenden Inhalationstherapievernebler beginnt die Einschaltphase des Aerosolerzeugers regelmäßig gleich zu Beginn einer Therapiesitzung und wird durch das Aktivieren der Aerosolerzeugung durch den Patienten (Einschalten des Verneblers) gestartet. Die Einschaltphase des Aerosolerzeugers endet, wenn der Patient die Aerosolerzeugung unterbricht (Ausschalten des Verneblers) oder wenn die zu vernebelnde Flüssigkeit verbraucht ist.

Bei einem durch die Atmung des Patienten gesteuerten Inhalationstherapievernebler wird als Einschaltphase jeweils der Zeitraum angesehen, der durch eine die Atmung des Patienten erfassende Steuereinrichtung festgelegt wird. Die Steuereinrichtung startet die Aerosolerzeugung (Einschalten des Aerosolerzeugers) zu einem durch die Atmung des Patienten festgelegten Zeitpunkt und unterbricht die Aerosolerzeugung (Ausschalten des Aerosolerzeugers) zu einem anderen durch die Atmung des Patienten festgelegten Zeitpunkt. Bei dieser Steuerung treten während einer Therapiesitzung mehrere aufeinanderfolgende Einschaltphasen auf, die auf die Atmung, üblicherweise die Einatemphasen, des Patienten abgestimmt sind.

In der Einschaltphasen bzw. den Einschaltphasen erzeugt der Aerosolgenerator ein Aerosol aus Flüssigkeitströpfchen, die dem Benutzer der Inhalationstherapievorrichtung als Aerosol zur Einatmung dargeboten werden.

Aerosolgeneratoren mit einer Membran und einer Schwingungserzeugungseinrichtung eignen sich in besonderem Maße für Inhalationstherapiezwecke, da das von diesen Aerosolgeneratoren erzeugte Aerosol von besonders hoher und gleichbleibender Qualität ist. Denn die erzeugten Flüssigkeitströpfchen liegen bei Membranaerosolgeneratoren zu einem sehr hohen Anteil in einer lungengängigen Größe von 0,1 bis 10 µm (MMD) und vorzugsweise von 0,5 bis 5 µm (MMD) vor. Überdies ist die Gesamtabgaberate (Total Output Rate = TOR) hoch, so dass Therapiesitzungen bei einem Inhalationstherapievernebler mit Membranaerosolgenerator in der Regel vergleichsweise kurz sind. In vielen Fällen ist dies vorteilhaft und gewünscht, da der Patient sich nur einer kurzen Therapiesitzung unterziehen muss. Jedoch besteht in anderen Fällen der Wunsch nach einer verlängerten Therapiesitzung, die aber nicht mit einer erhöhten Dosis einhergehen soll. In wieder anderen Fällen soll bei gleichbleibender Dauer der Therapiesitzungen die jeweils verabreichte Dosis unterschiedlich sein. In Anbetracht dieser grundsätzlichen Anforderungen wäre es wünschenswert, wenn die Menge der pro Zeiteinheit als Aerosol abgegebenen Flüssigkeit variabel reduziert werden könnte. Jedoch darf diese Beeinflussung der Abgaberate des Aerosolgenerators nicht mit einer Verschlechterung der Aerosolqualität einhergehen.

Auch andere Überlegungen sprechen für eine gezielte Reduzierung der Dosis des Medikamentenaerosols (TOR) bei einer Inhalationstherapie. Einige dieser Überlegungen sind im Folgenden zusammengefasst:
- Vermeidung einer Reizung (Überdosierung) der gesamten oder regionalen (lokalen) Atemwege, wie z.B.:
   o Reizung im Rachenraum und an der Glotis, die Husten verursachen können;
   o Lokale Überdosierung der Lunge, z.B.: an Aufzweigungen (Bifurkation) oder in bestimmten Bereichen der Atemwege (Trachea, Bronchien, Bronchiolen und Alveolen) oder möglichen Verengungen der Atemwege (Obstruktionen) und damit unerwünschte erhöhte Nebenwirkung bzw. Schädigung des Lungengewebes verursachen.
- Reduzierung der Medikamentenausbringrate (TOR), z.B.
   in der neonatalen Behandlung von Frühgeborenen mit kleinen Atemvolumina und hohen Atemfrequenzen, um eine genaue Applikation in den kleinen Lungen zu ermöglichen und eine möglichst effiziente Deposition des Wirkstoffs, wie z.B. teueres "Surfactant", zu erreichen.
- Vermeidung von lokalen Überreaktionen, Reizungen und Lungengewebeschädigungen im Zusammenhang mit Immunsuppressiva bei Lungentransplantationen. Mögliche Wirkstoffe sind Glucocorticoide, Zytostatika (Alkylantien. Antimetabolite, Interkalantien), Antikörper, veränderte Immunophilinen und weitere Substanzen.
- Bereitstellen einer adäquaten Medikamentendosis bei Untersuchungen im Rahmen von Zulassungen für Medikamente und Substanzen für Anwendungen bei Tieren für die generelle Behandlung beim Tierarzt oder für toxikologische Studien (TOX) ; speziell für die kleineren Tierlungen, wie Mäuse, Raten und Hunde. Eine Anwendung bei anderen Tieren, wie Pferden, ist ebenfalls möglich.
- Testen der Lunge eines Patienten auf
   Überempfindlichkeit des Bronchialsystems (hyperreaktives Bronchialsystem) bei unspezifischem oder spezifischem Provokationstest für die Lungendiagnose. Dabei wird z.B. Histamin, Methacholin oder ein Allergen mit steigender Dosis inhaliert. Die ersten Substanzdosen sind dabei oft sehr klein und sollten dosisgenau und sicher appliziert werden.
- Verabreichung neuer sehr potenter Medikamente aus Sicherheitsgründen und für erhöhte Dosisgenauigkeit mit einer längeren Inhalationstherapiedauer.
- Protektion von Personen die indirekt an der Inhalationstherapie beteiligt sind, wie Angehörigen, medizinisches Personal in Kliniken und Arztpraxen.

Vor diesem Hintergrund besteht das von der Erfindung zu lösende technische Problem darin, einen Inhalationstherapievernebler mit Membranaerosolgenerator zu schaffen, bei dem die Menge der pro Zeiteinheit als Aerosol abgegebenen Flüssigkeit beeinflusst werden kann, ohne dass die Qualität des Aerosols beeinträchtigt wird.

Dieses Problem wird gelöst durch eine Inhalationstherapievorrichtung mit den im Patentanspruch 1 angegebenen Merkmalen. Vorteilhafte Ausgestaltungen ergeben sich aus den Unteransprüchen.

Erfindungsgemäß wird das Ansteuersignal, das die Schwingungserzeugungseinrichtung des Aerosolgenerators zu Schwingungen anregt und bei dem es sich um eine Wechselspannung handelt, in den Einschaltphasen des Aerosolgenerators in vergleichsweise kurzen Intervallen zugeführt, die in kurzen Abständen aufeinander folgen. Die Werte für die Ansteuerphasen EIN/AUS liegen in den Bereichen von 10 ms bis 350 ms, vorzugseise 10 bis 150 ms, für die EIN-Phase und von 10 ms bis 500 ms, vorzugsweise 10 bis 250 ms, für die AUS-Phase. Diese intervallartige Zuführung des Ansteuersignals/der Wechselspannung und die Auswahl geeigneter Verhältnisse der EIN/AUS-Phasen währende der Einschaltphasen erlauben eine Beeinflussung der pro Zeiteinheit als Aerosol abgegebenen Flüssigkeitsmenge und damit der Gesamtabgaberate (Total Output Rate = TOR), in dem das Tastverhältnis der Intervalle verändert wird. Werden die Intervalle, in denen das Ansteuersignal/Wechselsignal zugeführt wird, verlängert und die Abstände zwischen den Intervallen verkürzt, erhöht sich die Abgaberate; werden die Intervalle verkürzt und die Abstände zwischen den Intervallen vergrößert, verringert sich die Abgaberate.

Dabei ist durchaus überraschend, dass die Aerosolqualität des von dem Membranaerosolgenerator abgegebenen Aerosols sich nicht verändert oder verschlechtert, obwohl der Aerosolgenerator in sehr kurzer Folge ein- und ausgeschaltet wird. Es konnte nicht erwartet werden, dass diese aus herkömmlicher Sicht unübliche Betriebsart des Aerosolerzeugers zu keiner Veränderung oder Verschlechterung des Tröpfchenspektrums (MMD, GSD), das heißt der Qualität des Aerosols, führt.

In Anbetracht dieses Umstands kann die erfindungsgemäße Lösung auch herangezogen werden, um fertigungsbedingte Schwankungen, zum Beispiel hinsichtlich der Löcheranzahl der Membran, zu kompensieren. Dabei wird eine konstante Substanzdosis bzw. TOR für alle Membranen einer Gerätespezifikation (Gerätekonfiguration angepasst an Medikament und Patientengruppe) eingestellt. Bei Gerätewechsel oder Wechsel des Aerosolgenerators könnte dies nötig werden, um neue dosiskritische Substanzen mit der gleichen Genauigkeit zu applizieren. Ein Gerätewechsel kann aus unterschiedlichen Gründen notwendig werden, mögliche Beispiele sind Gerätewartung, -reparatur, -defekt, -Zyklusende und Patiententransfer von Heim- zur Praxis- bzw. Klinikanwendung.

Zudem kann die erfindungsgemäße Lösung für den Ausgleich von Veränderungen herangezogen werden, die möglicherweise durch an der Membran auftretende Alterungsprozesse hervorgerufen werden. Diese Veränderungen können sich in Form einer absinkenden TOR über den Lebenszyklus einer Membran zeigen. Bei Anwendungen mit bekannten Alterungsprozessen kann mithilfe der Erfindung über die Lebensdauer des Aerosolgenerators eine konstante Substanzdosis eingestellt werden. Dafür wird am Anfang eine reduzierte TOR eingestellt und über die Zeit bzw. die Anzahl der Anwendungen wird diese TOR-Reduzierung mithilfe der Erfindung wieder ausgeglichen, sodass insgesamt eine konstante TOR über die gesamte Lebensspanne gegeben ist.

Im Folgenden wird die Erfindung anhand der Zeichnungen genauer erläutert, in denen zeigt:
- Figur 1: den grundsätzlichen Aufbau eines Membranaerosolgenerators;
- Figur 2A und 2B: die Einschaltphasen des Aerosolgenerators im kontinuierlichen und atemzuggesteuerten Betrieb;
- Figur 3: ein erfindungsgemäßes Ansteuersignal für den Aerosolgenerator;
- Figur 4: eine schematische Darstellung der relevanten Teile eines Ausführungsbeispiels eines erfindungsgemäßen Inhalationstherapieverneblers; und
- Figur 5: ein Diagramm, das Messwerte darstellt, die mit einem erfindungsgemäßen Aerosolgenerator erzielt wurden

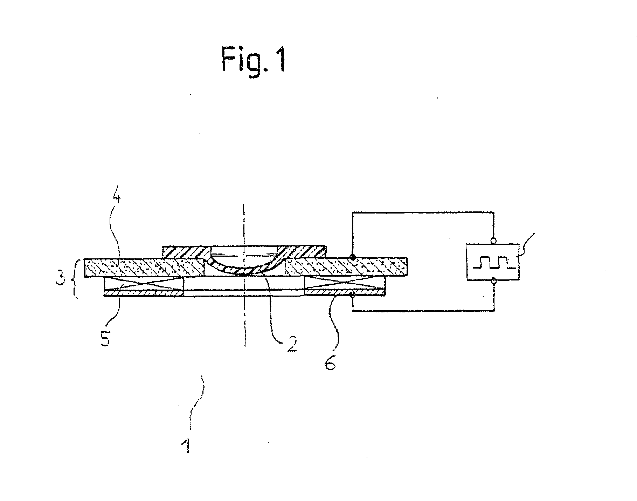

In Figur 1 ist zum besseren Verständnis der Erfindung beispielhaft der Aufbau des Aerosolgenerators 1 gezeigt, der eine Membran 2 und einen Schwingungserzeuger 3 umfasst. Die in Figur 1 gezeigte Membran ist gewölbt und weist im Bereich der Wölbung Öffnungen bzw. Löcher auf, durch die eine Flüssigkeit hindurchtritt, wenn die Membran in Schwingungen versetzt wird. Dazu ist die Membran mit dem Schwingungserzeuger 3 verbunden, der in dem in Figur 1 gezeigten Beispiel aus einem Substrat 4, einem Piezoelement 5 und einer Elektrode 6 aufgebaut ist. Über das leitfähige Substrat 4 und die Elektrode 6 wird dem Piezoelement 5 eine Wechselspannung zugeführt, die das aus Piezoelement und Substrat aufgebaute schwingungsfähige Gebilde zu Schwingungen anregt und die von einer Ansteuerschaltung 10 bereitgestellt wird. Die Schwingungen werden auf die an dem schwingungsfähigen Gebilde befestigte Membran übertragen, so dass auch die Membran in Schwingungen versetzt wird, wenn dem Piezoelement eine Wechselspannung zugeführt wird.

Die Frequenz der zugeführten Wechselspannung wird im wesentlichen bestimmt durch das Resonanzverhalten des Aerosolgenerators. Bei Aerosolgeneratoren, die für den Einsatz in Inhalationstherapieverneblern für die Erzeugung eines therapiegeeigneten Aerosols geeignet sind, werden regelmäßig Wechselsignale mit Frequenzen oberhalb von 100 kHz verwendet.

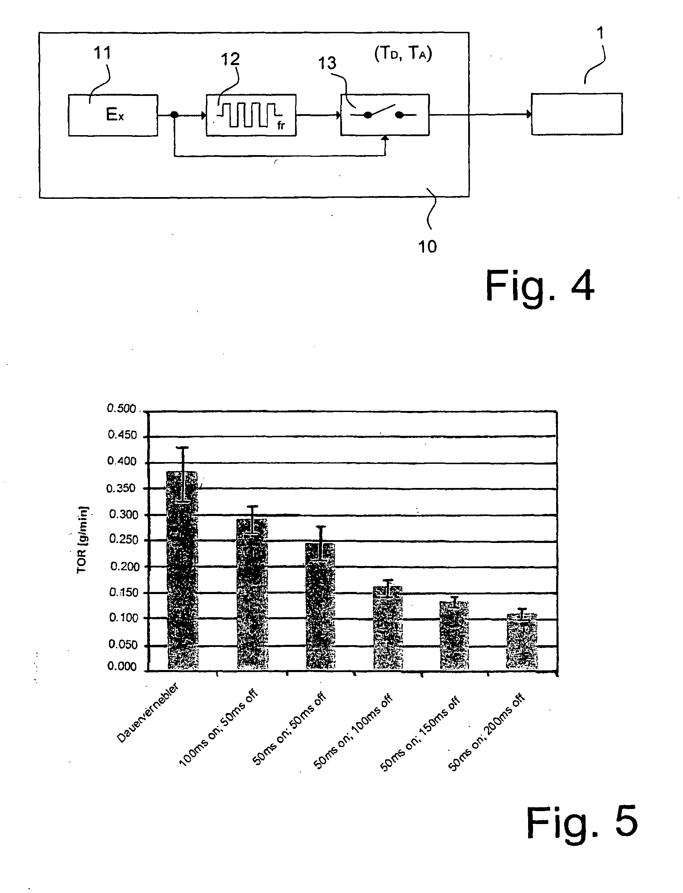

Erfindungsgemäß wird diese Wechselspannung in den Einschaltphasen des Aerosolgenerators nicht kontinuierlich sondern in mehreren aufeinanderfolgenden und voneinander beabstandeten Intervallen der Schwingungserzeugungseinrichtung des Aerosolgenerators zugeführt. Somit ist das erfindungsgemäße Ansteuersignal eine Wechselspannung, die für die Dauer der Einschaltphase(n) des Aerosolgenerators in mehreren Einzelintervallen in der Größenordnung von einigen Millisekunden, die voneinander einige Millisekunden beabstandet sind, dem Aerosolgenerator zur Ansteuerung zugeführt wird. Beispielhaft wurden in Versuchsreihen verschiedene Intervalle und Abstände zwischen den Intervallen untersucht und festgestellt, dass für Intervalle von 10 bis 150 ms in Abständen von 10 bis 250 ms eine präzise vorhersagbare, linear korrelierte Reduzierung der Gesamtabgaberate (Total Output Rate = TOR) bei gleichbleibend guten MMD-Werten (Aerosolqualität) erreicht werden kann. Beispielhaft zeigt Figur 5 die Ergebnisse von Messungen, die an 6 Membran-Aerosolgeneratoren durchgeführt wurden. Dabei wurde der Dauerbetrieb (Dauervernebler), bei dem die Zuführung des Ansteuersignals kontinuierlich erfolgte, in Gegenüberstellung zur erfindungsgemäßen Intervallansteuerung bei den in der Figur 5 angegebenen EIN/AUS-Intervallen (on/off) untersucht. Die Balken geben den Mittelwert über die 6 Aerosolgeneratoren an, wobei für jeden Mittelwert in Figur 5 auch die Standardabweichung dargestellt ist.

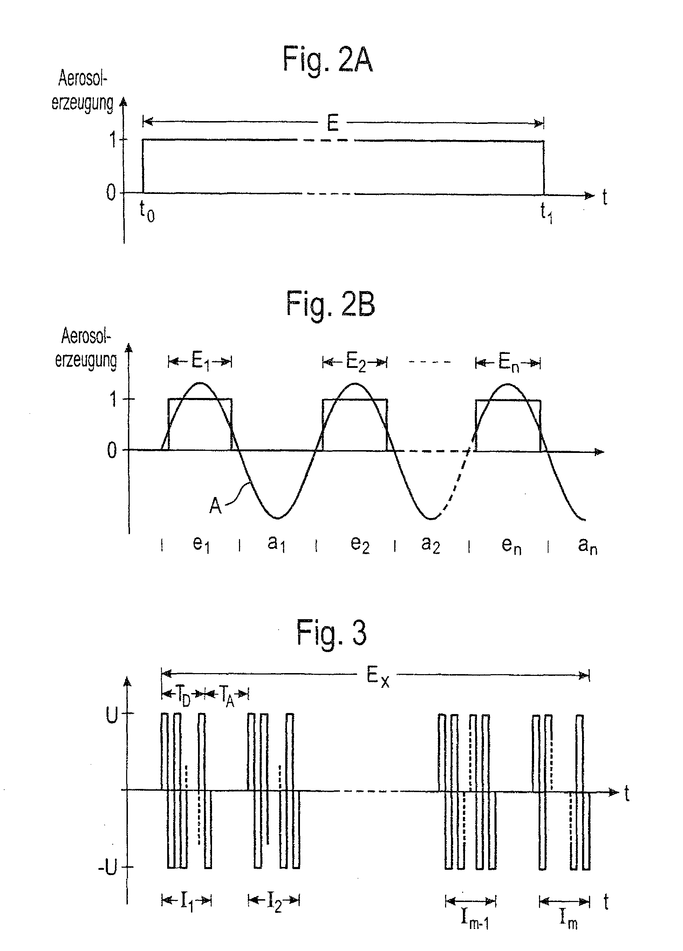

Figur 2A zeigt die Einschaltphase E des Aerosolgenerators bei einem Inhalationstherapievernebler, bei dem die Aerosolerzeugung kontinuierlich während der gesamten Therapiesitzung erfolgt. Nach dem Einschalten des Inhalationstherapieverneblers wird dem Aerosolgenerator das Ansteuersignal zugeführt, so dass zum Zeitpunkt to die Erzeugung des Aerosols beginnt und zu einem Zeitpunkt t₁ endet. Regelmäßig handelt es sich dabei um den Zeitpunkt, bei dem die zu vernebelnde Flüssigkeit aufgebraucht ist. Jedoch kann der Patient die Therapiesitzung auch von sich aus beenden, indem er den Inhalationstherapievernebler ausschaltet. Bei kontinuierlicher Aerosolerzeugung ist die Einschaltphase E unabhängig von der Atmung des Patienten.

Im Vergleich dazu zeigt Figur 2B den Fall eines Inhalationstherapieverneblers mit einer atemzuggesteuerten Aerosolerzeugung. Wie der Figur 2B zu entnehmen ist, sorgt eine (nicht dargestellte) Steuervorrichtung dafür, dass in Abhängigkeit von der Atmung des Patienten, deren Verlauf durch die Kurve A in Figur 2B angedeutet ist, mehrere Einschaltphasen E₁ ... Eₙ erzeugt werden. Dabei zeigt Figur 2B den üblichen Fall, dass die Einschaltphasen E₁ ... Eₙ in Abstimmung mit den Einatemphasen e₁...eₙ auftreten, während in den Ausatemphasen a₁...aₙ der Aerosolgenerator nicht eingeschaltet ist.

Figur 3 zeigt das erfindungsgemäße Ansteuersignal anhand der beispielhaften Einschaltphase Eₓ, bei der es sich entweder um die Einschaltphase E gemäß Figur 2A oder eine der Einschaltphasen E₁ ... Eₙ gemäß Figur 2B handelt. Dabei ist zu beachten, dass die Darstellung in Figur 3 nur schematisch ist, um den Charakter des erfindungsgemäßen Ansteuersignals für den Aerosolgenerator zu verdeutlichen. Insbesondere ist das erfindungsgemäße Ansteuersignal nicht auf das in der Figur 3 gezeigte rechteckförmige Wechselsignal beschränkt. Aus Figur 3 ist zu entnahmen, dass in der Einschaltphase Eₓ dem Aerosolgenerator eine Wechselspannung in mehreren aufeinanderfolgenden Intervallen I₁...Iₘ zugeführt wird, die voneinander beabstandet sind. Letztlich wird erfindungsgemäß nur in den Intervallen I₁...Iₘ die Schwingungserzeugungseinrichtung und damit die Membran des Aerosolgenerators zu Schwingungen angeregt, so dass auch nur in diesen Intervallen eine Aerosolabgabe erfolgt. Durch die Anzahl der Intervalle während einer Einschaltphase Eₓ kann die in der Einschaltphasen Eₓ abgegebene Aerosolmenge und damit die Gesamtabgaberate (Total Output Rate = TOR) des Inhalationstherapieverneblers beeinflusst werden.

In Figur 3 ist gezeigt, dass die Intervalle I₁...Iₘ die Länge T_{D} und die Abstände zwischen ihnen die Länge T_{A} besitzen. Über eine Veränderung der Werte T_{D} und T_{A} kann die Abgaberate verändert werden. In Figur 3 ist angedeutet, dass die Werte T_{D} und T_{A} für eine Einschaltphase Ex konstant sind. Alternativ können die Werte T_{D} und T_{A} aber auch innerhalb einer Einschaltphase Ex verändert werden.

Figur 4 zeigt schematisch die hier relevanten Komponenten eines Inhalationstherapieverneblers gemäß einem Ausführungsbeispiel der Erfindung.

Mit der Bezugsziffer 1 ist der Aerosolgenerator bezeichnet, dessen Aufbau sich im Einzelnen aus Figur 1 ergibt. Insofern wird auf die Figur 1 Bezug genommen.

Dem Aerosolgenerator 1 wird von einer Ansteuereinrichtung 10 eine Wechselspannung, zugeführt, die die Schwingungseinrichtung und damit die Membran des Aerosolgenerators 1 in Schwingungen versetzt, so dass ein Aerosol erzeugt wird. Erfindungsgemäß führt die Ansteuereinrichtung 10 die Wechselspannung in mehreren aufeinanderfolgenden und voneinander beabstandeten Intervallen in den Einschaltphasen des Aerosolgenerators zu. Dazu umfasst die Ansteuereinrichtung bei dem in Figur 4 gezeigten Ausführungsbeispiel eine Einschaltphasensteuereinrichtung 11, die die Einschaltphasen Eₓ für den kontinuierlichen Betrieb (Figur 2A) oder den atemzuggesteuerten Betrieb (Figur 2B) festlegt. Während dieser Einschaltphasen erzeugt ein Wechselspannungsgenerator 12 ein Ansteuersignal, zum Beispiel eine rechteckförmige oder sinusförmige (oder andere geeignete) Wechselspannung, deren Frequenz fr auf den Aerosolgenerator 1 abgestimmt ist. Das Ausgangssignal des Wechselspannungsgenerators 12 wird einer Intervallschalteinrichtung 13 zugeführt, die während einer Einschaltphase Eₓ die Intervalle und den Abstand zwischen ihnen bestimmt, in denen das Wechselspannungssignal des Wechselspannungsgenerators 12 dem Aerosolgenerator 1 zugeführt wird. Die Intervallschalteinrichtung 13 erhält dazu Vorgabewerte für die Dauer der Intervalle T_{D} und den Abstand zwischen den Intervallen T_{A}. Während der Einschaltphase Eₓ schaltet die Intervallschalteinrichtung 13 periodisch das Wechselspannungssignal des Wechselspannungsgenerators 12 während einer Zeitspanne T_{D} zum Aerosolgenerator 1 durch, so dass in diesen Intervallen der Aerosolgenerator ein Aerosol abgibt. Daran anschließend wird für eine Zeitspanne T_{A} die Weiterleitung des Wechselspannungssignals von dem Wechselspannungsgenerator 12 zum Aerosolgenerator 1 unterbrochen. In dieser Zeit erzeugt der Aerosolgenerator kein Aerosol. Diese Weiterleitung des Wechselspannungssignals von dem Wechselspannungsgenerator 12 über die Intervallschalteinrichtung 13 zum Aerosolgenerator 1 jeweils für einen Zeitraum T_{D} wiederholt sich periodisch während der gesamten Einschaltphase Ex.

Durch die Einstellung der Intervalllänge T_{D} und der Intervallabstände T_{A} kann die Aerosolmenge festgelegt werden, die von dem Aerosolgenerator 1 während einer Einschaltphase Eₓ abgegeben wird. Dabei kann vorgesehen werden, dass die Vorgabewerte für die Intervallschalteinrichtung 13 stufenlos wählbar/einstellbar bzw. aus einer Speichervorrichtung abrufbar sind.

## Patentansprüche

1. Inhalationstherapievorrichtung mit
- einem Aerosolgenerator (1), der
- eine Membran (2), die aus einer auf der einen Seite anstehenden Flüssigkeit Flüssigkeitströpfchen erzeugt und auf der anderen Seite als Aerosol abgibt, wenn die Membran in Schwingungen versetzt wird, und
- eine Schwingungserzeugungseinrichtung (3) umfasst, die mit der Membran derart verbunden ist, dass die Membran in Schwingungen versetzt wird, wenn die Schwingungserzeugungseinrichtung in den Einschaltphasen des Aerosolgenerators durch ein zugeführtes Ansteuersignal zu Schwingungen angeregt wird, und
- einer Ansteuereinrichtung (10), die mit der
Schwingungserzeugungseinrichtung verbunden ist, wobei
die Ansteuereinrichtung (10) dafür ausgelegt ist, der Schwingungserzeugungseinrichtung (3) in den Einschaltphasen (Eₓ) des Aerosolgenerators (1) eine wechselspannung in mehreren aufeinander folgenden und voneinander beabstandeten Intervallen (I₁...Iₙ) zuzuführen,
**dadurch gekennzeichnet, dass**
die Ansteuereinrichtung (10) einen Wechselspannungsgenerator (12) und eine Intervallschalteinrichtung (13) aufweist, der die von dem Wechselspannungsgenerator erzeugte Wechselspannung zugeführt wird und die die Wechselspannung in den Intervallen (I₁...Iₙ) an den Aerosolgenerator (1) weiterleitet, und
dass der Intervallschalteinriehtung (13) die Dauer (T_{D}) der Intervalle und die Abstände (T_{A}) zwischen den Intervallen (I₁...Iₙ) zugeführt werden und die Dauer (T_{D}) im Bereich von 10 ms bis 350 ms, vorzugsweise 10 bis 150 ms und die Abstände (T_{A}) im Bereich von 10 ms bis 500 ms, vorzugsweise 10 bis 250 ms liegen.

2. Inhalationstherapievorrichtung nach Anspruch 1, **dadurch gekennzeichnet, dass** die Dauer (T_{D}) und die Abstände (T_{A}) variabel sind, insbesondere stufenlos einstellbar sind.

3. Inhalationstherapievorrichtung nach einem der Ansprüche 1 bis 2, **dadurch gekennzeichnet, dass** die Ansteuereinrichtung (10) eine Einschaltphasensteuereinrichtung (11) umfasst, die die Einschaltphasen des Aerosolgenerators (1) festlegt.

4. Inhalationstherapievorrichtung nach Anspruch 3, **dadurch gekennzeichnet, dass** die Einschaltphasensteuereinrichtung (11) eine kontinuierliche Einschaltphase (E) festlegt.

5. Inhalationstherapievorrichtung nach Anspruch 3, **dadurch gekennzeichnet, dass** die
Einschaltphasensteuereinrichtung (11) atemzuggesteuert mehrere Einschaltphase (E₁... Eₙ) festlegt.

6. Inhalationstherapievorrichtung nach einem der Ansprüche 1 bis 5, **dadurch gekennzeichnet, dass** die Schwingungserzeugungseinrichtung (3) ein Substrat (4) und ein Piezoelement (5) umfasst.

## Claims

1. Inhalation treatment device having
- an aerosol generator (1) which comprises
- a membrane (2) which generates liquid droplets from a liquid present on the one side and releases them as aerosol on the other side when the membrane is vibrated, and
- a vibration-generating device (3) which is connected to the membrane such that the membrane is vibrated when the vibration-generating device is vibrated in the switch-on phases of the aerosol generator by a supplied activation signal, and
- an activation device (10) which is connected to the vibration-generating device, wherein the activation device (10) is designed for supplying an alternating voltage in several sequential intervals (I₁...Iₙ) spaced from one another to the vibration-generating device (3) in the switch-on phases (Eₓ) of the aerosol generator (1), **characterised in that** the activation device (10) has an alternating-voltage generator (12) and an interval switching device (13), to which the alternating voltage generated by the alternating-voltage generator is supplied and which forwards the alternating voltage in the intervals (I₁...Iₙ) to the aerosol generator (1), and **in that** the length (T_{D}) of the intervals and the distances (T_{A}) between the intervals (I₁...Iₙ) are supplied to the interval switching device (13) and the length (T_{D}) lies in the range from 10 ms to 350 ms, preferably 10 to 150 ms and the distances (T_{A}) lie in the range from 10 ms to 500 ms, preferably 10 to 250 ms.

2. Inhalation treatment device according to claim 1, **characterised in that** the length (T_{D}) and the distances (T_{A}) are variable, in particular are infinitely adjustable.

3. Inhalation treatment device according to one of claims 1 to 2, **characterised in that** the activation device (10) comprises a switch-on phase control device (11) which determines the switch-on phases of the aerosol generator (1).

4. Inhalation treatment device according to claim 3, **characterised in that** the switch-on phase control device (11) determines a continuous switch-on phase (E).

5. Inhalation treatment device according to claim 3, **characterised in that** the switch-on phase control device (11) determines several switch-on phases (E₁...Eₙ) in breath-controlled manner.

6. Inhalation treatment device according to one of claims 1 to 5, **characterised in that** the vibration-generating device (3) comprises a substrate (4) and a piezoelement (5).

## Revendications

1. Dispositif de thérapie par inhalation, avec :
- un générateur d'aérosol (1), comprenant
- une membrane (2), laquelle génère des gouttelettes de liquide à partir d'un liquide arrivant d'un premier côté et refoulant celles-ci sous forme d'aérosol sur le deuxième côté quand la membrane est soumise à oscillations, et
- un dispositif de génération d'oscillations (3), lequel est relié à la membrane de manière à soumettre la membrane à oscillations quand le dispositif de génération d'oscillations est excité à osciller par un signal de commande appliqué pendant les phases de démarrage du générateur d'aérosol, et
- un dispositif de commande (10) relié au dispositif de génération d'oscillations,
le dispositif de commande (10) étant prévu pour délivrer au dispositif de génération d'oscillations (3) un signal de commande, en particulier une tension alternative, à plusieurs intervalles (I₁ ... Iₙ) successifs et espacés l'un de l'autre pendant les phases de démarrage (Eₓ) du générateur d'aérosol (1),
**caractérisé en ce que**
le dispositif de commande (10) comporte un générateur de tension alternative (12) et un dispositif de commutation d'intervalle (13) auquel est appliquée la tension alternative générée par le générateur de tension alternative et qui transmet au générateur d'aérosol (1) la tension alternative pendant les intervalles (I₁ ... Iₙ), et
la durée (T_{D}) des intervalles et les écarts (T_{A}) entre intervalles (I₁ ... Iₙ) sont délivrés au dispositif de commutation d'intervalle (13), et **en ce que** la durée (T_{D}) est comprise dans une plage de 10 ms à 350 ms, préférentiellement de 10 à 150 ms, et les écarts (T_{A}) dans une plage de 10 ms à 500 ms, préférentiellement de 10 à 250 ms.

2. Dispositif de thérapie par inhalation selon la revendication 1, **caractérisé en ce que** la durée (T_{D}) et les écarts (T_{A}) sont variables, et réglables en particulier de manière continue.

3. Dispositif de thérapie par inhalation selon l'une des revendications 1 et 2, **caractérisé en ce que** le dispositif de commande (10) comprend un dispositif de commande des phases de démarrage (11) qui définit les phases de démarrage du générateur d'aérosol (1).

4. Dispositif de thérapie par inhalation selon la revendication 3, **caractérisé en ce que** le dispositif de commande des phases de démarrage (11) définit une phase de démarrage (E) continue.

5. Dispositif de thérapie par inhalation selon la revendication 3, **caractérisé en ce que** le dispositif de commande des phases de démarrage (11) définit plusieurs phases de démarrage (E₁ ... Eₙ) commandées par inspiration.

6. Dispositif de thérapie par inhalation selon l'une des revendications 1 à 5, **caractérisé en ce que** le dispositif de génération d'oscillations (3) comprend un substrat (4) et un élément piézo-électrique (5).
